# EUROPÄISCHE PATENTSCHRIFT

(11) **EP 1 685 577 B1**
(45) Veröffentlichungstag und Bekanntmachung des Hinweises auf die Patenterteilung: **05.09.2012**
(21) Anmeldenummer: 04791022.9
(22) Anmeldetag: 29.10.2004
(51) Int. Cl.: H01H 3/14

(54) **STEUEREINRICHTUNG ZUR STEUERUNG ELEKTROMEDIZINISCHER GERÄTE**
CONTROL DEVICE FOR CONTROLLING ELECTROMEDICAL DEVICES
DISPOSITIF DE COMMANDE POUR COMMANDER DES APPAREILS ELECTROMEDICAUX

(30) Priorität: 03.11.2003 DE 10351199
(43) Veröffentlichungstag der Anmeldung: 02.08.2006
(73) Patentinhaber: ERBE Elektromedizin GmbH, 72072 Tübingen (DE)
(72) Erfinder: KÜHNER, Ralf, 70567 Stuttgart (DE); HUNDT, Roland, 72072 Tübingen (DE)
(74) Vertreter: Bohnenberger, Johannes
(86) Internationale Anmeldenummer: PCT/EP2004/012261
(87) Internationale Veröffentlichungsnummer: WO 2005/043569

(56) Entgegenhaltungen:
- EP-A2- 1 217 640
- WO-A1-02/32354
- DE-U- 20 312 016
- US-A- 6 017 354
- US-A- 6 051 797

## Beschreibung

Die Erfindung betrifft eine Steuereinrichtung zur Steuerung elektromedizinischer Geräte oder Gerätegruppen.

Zur Steuerung elektromedizinischer Geräte, z.B. HF-Generatoren, Laser-Geräte, Wasserstrahl-chirurgischer Geräte oder dergleichen, die insbesondere im Operationssaal Verwendung finden, sind Schalteinrichtungen notwendig, die insbesondere vom Operateur verwendet werden, um die Funktion der angeschlossenen Geräte oder Gerätegruppen entsprechend den Bedürfnissen zu steuern. Beispielsweise wird zum Stillen einer Blutung ein APC-Gerät (Argon-Plasma-Koagulation) verwendet, bei welchem durch Betätigung eines Fußschalters einerseits ein Ventil geöffnet wird, um Argon einer Sonde (und damit der Anwendungsstelle) zuzuführen und andererseits eine HF-Generator so anzusteuern, dass ein hochfrequenter Strom von einer Elektrode in der Sonde unter Ionisierung des Argon zum Gewebe fließt, wodurch dieses koaguliert wird. Mit demselben Gerät kann auch eine andere Funktion durchgeführt werden, zu welcher der HF-Generator in eine andere Betriebsweise umgeschaltet wird. Alle diese Steuerfunktionen können mit einem, zumeist aber mit mehreren Schalteinrichtungen angesteuert werden, die oftmals als Taster oder Fußschalter ausgeführt sind. Wenn mehrere derartiger Taster oder Fußschalter zur Steuerung von einem oder mehren Geräten oder Gerätegruppen benötigt werden, so werden diese abgeschlossenen Systeme jeweils über ein Kabel mit den entsprechenden Geräten oder Gerätegruppen verbunden. Auch kabellose Verbindungen sind bekannt, jedoch wird auch bei diesen jeder Schalteinrichtung eine gesonderte Übertragungsstrecke zugeordnet. Soll an einem Gerät oder einer Gerätegruppe eine andere Funktion aktiviert werden können, so muss die entsprechende Schalteinrichtung zur Unterstützung der nun gewünschten Funktion neu eingesetzt oder eine andere Schalteinrichtung entsprechend umgeändert und umgesteckt werden. Die ist aufwändig.

Weiterhin besteht durch die unter Umständen notwendige Vielzahl von Kabelverbindungen eine erhebliche Störung des im Vordergrund stehenden Operationsbetriebes und zwar sowohl aufgrund mechanischer Störungen ("Stolperfallen") als auch aufgrund der von den vielen Kabeln und Steuerleitungen, die allesamt als Antennen wirken, verursachten elektromagnetischen Störungen. Die Vielzahl von Kabeln durch eine Vielzahl von Funkverbindungen zu ersetzen, führt wiederum zu einer Vielzahl von elektromagnetischen Störungen.

Ein aus mehreren Modulen zusammensetzbaren Fußselaleter ist aus EP 1217640 bekannt.

Der Erfindung liegt die Aufgabe zu Grunde, eine Steuereinrichtung der eingangs beschriebenen Art dahin gehend weiterzubilden, dass bei einem kompakten und störungsarmen Aufbau eine erhöhte Vielseitigkeit erzielbar ist.

Diese Aufgabe wird durch eine Steuereinrichtung zur Steuerung elektromedizinischer Geräte oder Gerätegruppen gemäß Anspruch 1 gelöst, umfassend mindestens zwei Fußschalter oder dergleichen Schalteinrichtungen zur Erzeugung von Steuersignalen zum Steuern der Geräte oder Gerätegruppen, Verbindungseinrichtungen an den Schalteinrichtungen, über welche die Schalteinrichtungen miteinander verbindbar sind, Zuweisungseinrichtungen, um den Steuersignalen bestimmte Steuerfunktionen bezüglich der Geräte oder Gerätegruppen zuzuweisen und Informationsübertragungseinrichtungen zum Übertragen der Steuersignale von den Schalteinrichtungen zu den Geräten oder Gerätegruppen.

Ein wesentlicher Punkt der Erfindung liegt darin, dass die Schalteinrichtungen nicht mehr direkt (und sei es auch über eine irgendwie geartete drahtlose Verbindung) mit den Geräten oder Gerätegruppen verbunden sind, vielmehr werden die Schalteinrichtungen indirekt über die Zuweisungseinrichtungen mit den Geräten oder Gerätegruppen verbunden, so dass dort jede Schalteinrichtung eine bestimmte, auf bestimmte Funktionen der Geräte oder Gerätegruppen zugeschnittene Steuerfunktion zugewiesen werden kann. Es kann somit ohne die Schalteinrichtungen an sich zu verändern sozusagen der Sinngehalt der durch sie erzeugten Signale verändert werden. Dadurch ist nicht nur eine Veränderung der Funktion bereits installierter Steuereinrichtungen möglich, es können vielmehr beliebig viele Steuereinrichtungen miteinander verkoppelt werden, so dass sozusagen eine "Klaviatur" entsteht, deren "Tastenbedeutung" vom Operateur entsprechend seinen Bedürfnissen festgelegt werden kann.

Die Verbindungseinrichtungen umfassen einen Datenbus, der derart ausgebildet ist, dass nach Verbinden einer Vielzahl von Schalteinrichtungen miteinander die Steuersignale aller Schalteinrichtungen an dem Datenbus zur Verfügung stehen. Dadurch wird in einfacher Weise gewährleistet, dass alle Signale der Schalteinrichtung gleichzeitig zur Verfügung stehen.

Jede der Schalteinrichtungen weist hierbei zwei mit dem Datenbus verbundene Steckverbinder derart auf, dass bei einer Reihe von miteinander verbundenen Schalteinrichtungen der Datenbus an mindestens einem der Stechverbinder mit den Informationsübertragungseinrichtungen verbindbar ist. Dadurch ist eine unbegrenzte Anzahl von Schalteinrichtungen zu einer "Klaviatur" zusammensteckbar.

Die Informationsübertragungseinrichtungen können mit den Geräten oder Gerätegruppen über eine Leitung verbunden werden. Alternativ ist es möglich, die Informationsübertragungseinrichtungen mit mindestens einem, mit den Schalteinrichtungen verbindbaren Sendern und mindestens einem mit den Geräten oder Gerätegruppen verbundenen Empfänger zur drahtlosen Übertragung der Steuersignale auszurüsten. Dadurch fallen die "Stolperfallen" weg.

Wie oben beschrieben können die Schalteinrichtungen direkt miteinander verbunden werden. Vorzugsweise sind Kodierungseinrichtungen zur Identifikation von Schalteinrichtungen durch die Geräte oder Gerätegruppen vorgesehen, so dass die Geräte die ihnen zugeordneten Schalteinrichtungen "selbst erkennen" und Fehlfunktionen oder -kombinationen ausgeschlossen werden können.

Nachfolgend wird die Erfindung anhand von Ausführungsbeispielen näher erläutert. Hierbei zeigen
- Fig. 1: eine Gruppe von Fußschaltern voneinander getrennt;
- Fig. 2: die Gruppe von Schaltern nach Fig. 1 in zusammengestecktem Zustand;
- Fig. 3: eine Gruppe von Fußschaltern mit einer Sockelplatte (nicht Teil ein beanspruchten Erfindung),
- Fig. 4: eine Gruppe von Fußschaltern mit drahtloser Verbindung und
- Fig. 5: ein schematisiertes Blockschaltbild einer Ausführungsform der erfindungsgemäßen Steuereinrichtung.

In der nachfolgenden Beschreibung werden für gleiche und gleich wirkende Teile dieselben Bezugsziffern verwendet.

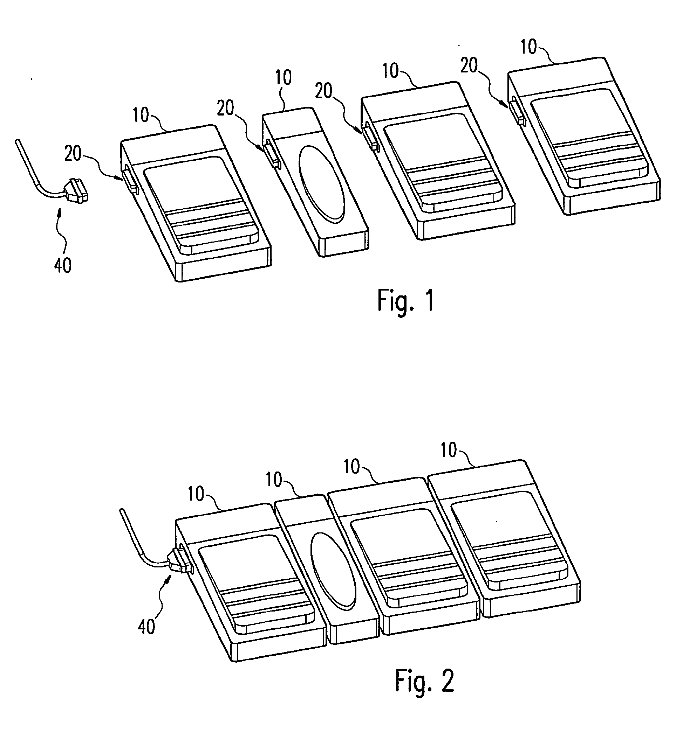

Wie aus Fig. 1 hervorgeht, wird eine Gruppe von Fußschaltern 10 vorgesehen, die jeweils eine mehrpolige Verbindungseinrichtung 20 und (auf der gegenüberliegenden Gehäuseseite, nicht gezeigt) eine korrespondierende Verbindungseinrichtung aufweisen, so dass die hier als Fußschalter ausgebildeten Schalteinrichtungen 10 zu einer Gruppe zusammengesteckt werden können, wie diese in Fig. 2 gezeigt ist. Darüber hinaus sind mechanische Verbindungen derart vorgesehen, dass die in Fig. 2 gezeigte Gruppe aus insgesamt vier Fußschaltern mechanisch fest miteinander verbunden ist und einheitlich gehandhabt werden kann. Zur Verbindung mit den steuernden Geräten, die weiter unten näher beschrieben werden, ist als Informationsübertragungseinrichtung 40 ein Kabel vorgesehen.

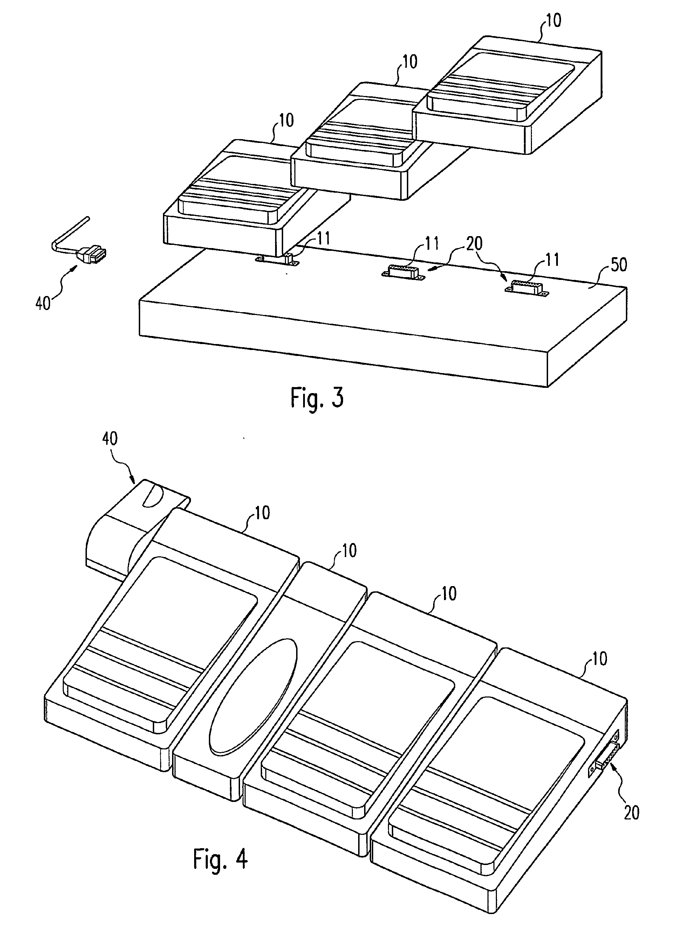

Das in Fig. 3 gezeigte nicht beanspruchle Beispiel unterscheidet sich von der nach Fig. 2 dadurch, dass die Fußschalter 10 nicht direkt miteinander sondern indirekt über entsprechende Steckverbinder 11 in einer Grundplatte 50 miteinander verbunden werden, wobei diese Grundplatte 50 gleichzeitig als Sockel für die Fußschaltergruppe dient. Die Verbindung zu den zu steuernden Geräten erfolgt auch hier wieder über ein Kabel 40.

Die in Fig. 4 gezeigte Ausführungsform unterscheidet sich von der nach den Fig. 1 und 2 dadurch, dass anstelle eines Kabels die Informationsübertragungseinrichtung 40 als drahtlose Übertragungseinrichtung ausgebildet ist, also als Funkübertragungseinrichtung oder als Infrarotübertragungseinrichtung. Hierbei wird in diese Übertragungseinrichtung auch eine wiederaufladbare Stromquelle vorgesehen, so dass die gesamte Einheit leicht transportabel und an beliebigen Orten aufstellbar ist, ohne eine Kabelzuführung zu benötigen. Selbstverständlich ist diese Ausführungsform auch mit dem Beispiel nach Fig. 3 kombinierbar. Insbesondere kann beispielsweise die drahtlose Informationsübertragungseinrichtung in der Grundplatte 50 vorgesehen sein.

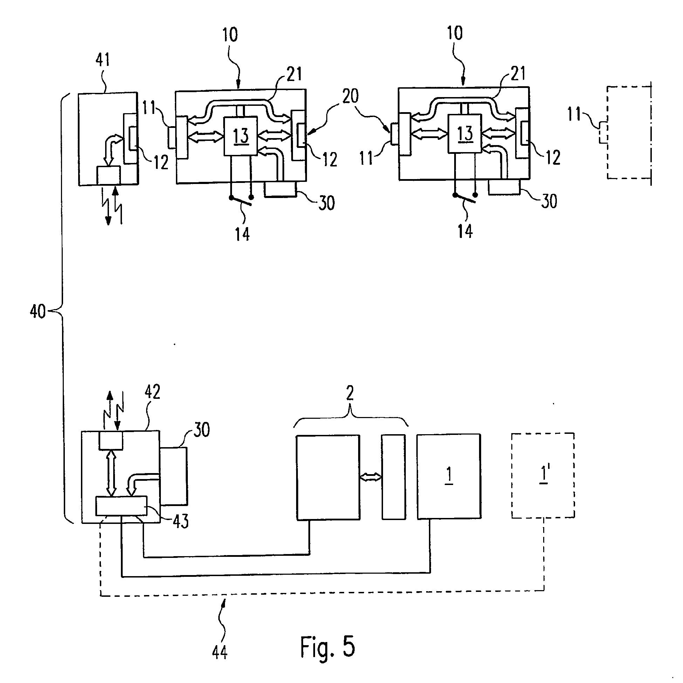

In Fig. 5 ist der elektrische Aufbau der Gesamtanordnung in einem prinzipiellen Blockschaltbild gezeigt. Aus dieser Abbildung geht hervor, dass jede der Schalteinrichtungen 10 Steckverbinder 11 und 12 aufweist, die derart korrespondierend geformt sind, dass beliebig viele Schalteinrichtungen 10 aneinander angereiht und miteinander elektrisch verbunden werden können. An die jeweils letzte (in Fig. 5 linke) Schalteinrichtung 10 kann dann die Informationsübertragungseinrichtung 40 angesteckt werden.

In jeder der Schalteinrichtungen 10 ist zum einen ein Datenbus 21 vorgesehen, der zum einen die am Steckverbinder 12 anstehenden Informationen einer weiteren angesteckten Schalteinrichtung 10 an den Steckverbinder 11 (und schließlich an die Informationsübertragungseinrichtung 40) weitergibt und der andererseits derart mit einer Signalerzeugungseinrichtung 13 verbunden ist, dass über einen Schalter 14, z.B. ein Pedal, wie in den Fig. 1 - 4 gezeigt, Schaltsignale erzeugt und an den Steckverbinder 11 weitergegeben werden. An den Schalteinrichtungen 10 sind weiterhin Zuweisungseinrichtungen 30 z.B. in Form von Kodierschaltern vorgesehen, über welche der jeweiligen Schalteinrichtungen 10 bestimmte Funktionen zugeordnet werden können in Bezug auf zu steuernde Geräte 1, 1' oder Gerätegruppen 2, die über die Schalteinrichtungen 10 gesteuert werden sollen.

Die im Datenbus 21 übertragenen Informationen werden über die Steckverbinder 11 und 12 einem Sender 41 zugeleitet, dessen Sendesignale von einem Empfänger 42 empfangen werden, der zusammen mit dem Sender 41 die Informationsübertragungseinrichtung 40 darstellt. Es sei darauf hingewiesen, dass anstelle einer derartigen drahtlosen. Übertragung auch ein Kabel verwendet werden kann.

Im Empfänger 42 kann auch die oben bereits erwähnte Zuweisungseinrichtung 30 vorgesehen sein, so dass jede Schalteinrichtung 10 der "Sinngehalt" einer von ihr erzeugten Schaltinformation auch an einer zentralen Stelle zugewiesen werden kann.

Weiterhin ist im Empfänger 42 eine Kodiereinrichtung 43 vorgesehen, die derart ausgebildet ist, dass die aus dem Empfänger 42 über Anschlusskabel 44 zu den Geräten 1, 1' oder Gerätegruppen 2 geführten Signalen nur die Steuerungsfunktionen ausführen können, die in den jeweiligen Geräten oder Gerätegruppen zulässig sind. Gleichfalls ist es möglich, durch derartige Kodierungen bestimmte Kombinationen von Funktionen zu sperren, so dass beispielsweise nicht gleichzeitig bei einer Operation mit ein und demselben Gerät gespült und dabei hochfrequenter Strom zugeleitet wird, der nur in Kombination mit der Zufuhr von Edelgas ohne Anwesenheit von Spülflüssigkeit eingeschaltet werden dürfte. Es kann also hierdurch auch gleichzeitig eine Fehlbedienung über die Schalteinrichtungen 10 ausgeschlossen werden.

Aus Obigem geht hervor, dass ein wesentlicher Punkt der Erfindung darin liegt, dass die Schalteinrichtungen 10 zunächst Funktionsunbestimmt sind und die Funktionszuweisung (einschließlich einer Absicherung gegen Fehlfunktionen) über die Zuweisungseinrichtungen 30 und die Kodiereinrichtungen 43 (zentral oder an jeder Schalteinrichtung 10) bewirkt werden.

### Bezugszeichenliste

- 1, 1': Gerät
- 2: Gerätegruppe
- 10: Schalteinrichtung
- 11: Steckverbinder
- 12: Steckverbinder
- 13: Signalerzeugungseinrichtung
- 14: Schalter
- 20: Verbindungseinrichtung
- 21: Datenbus
- 30: Zuweisungseinrichtung
- 40: Informationsübertragungseinrichtung
- 41: Sender
- 42: Empfänger
- 43: Kodiereinrichtung
- 44: Anschlusskabel
- 50: Grundplatte

## Patentansprüche

1. Steuereinrichtung zur Steuerung elektromedizinischer Geräte (1, 1') oder Gerätegruppen (2), umfassend:
- mindestens zwei Fußschalter (10) zur Erzeugung von Steuersignalen zum Steuern der Geräte (1, 1') oder Gerätegruppen (2),
- Steckverbinder (11, 12) an den Fußschaltern (10), über welche die Fußschalter (10) miteinander verbindbar sind,
- Zuweisungseinrichtungen (30), um den Steuersignalen bestimmte Steuerfunktionen bezüglich der Geräte (1, 1') oder Gerätegruppen (2) zuzuweisen, und
- Informationsübertragungseinrichtungen (40) zum Übertragen der Steuersignale von den Fußschaltern (10) zu den Geräten (1, 1') oder Gerätegruppen (2),
**dadurch gekennzeichnet, dass**
die Steckverbinder (11, 12) einen Datenbus (21) umfassen, der derart ausgebildet ist, dass nach Verbinden einer Vielzahl von Fußschaltern (10) miteinander die Steuersignale aller Fußschalter (10) an dem Datenbus (21) zur Verfügung stehen, wobei
jeder der Fußschalter (10) zwei mit dem Datenbus (21) verbundene Steckverbinder (11, 12) derart aufweist, dass bei einer Reihe von miteinander verbundenen Fußschaltern (10) der Datenbus (21) an mindestens einem der Steckverbinder (11, 12) mit den Informationsübertragungseinrichtungen (40) verbindbar ist.

2. Steuereinrichtung nach Anspruch 1,
**dadurch gekennzeichnet, dass**
die Informationsübertragungseinrichtungen (40) mindestens einen mit den Fußschaltern (10) verbindbaren Sender (41) und mindestens einen mit den Geräten (1, 1') der Gerätegruppen (2) verbundenen Empfänger (42) zur drahtlosen Übertragung der Steuersignale umfassen.

3. Steuereinrichtung nach einem der vorhergehenden Ansprüche,
**gekennzeichnet durch**
Kodiereinrichtungen (43) zur Identifikation von Fußschaltern (10) **durch** die Geräte (1, 1') oder Gerätegruppen (2).

## Claims

1. Control device for controlling electromedical devices (1, 1') or device groups (2), comprising:
- at least two foot-operated switches (10) for generating control signals for controlling the devices (1, 1') or device groups (2),
- plug connectors (11, 12) on the foot-operated switches (10), it being possible to connect the foot-operated switches (10) to one another by means of the said plug connectors,
- assignment devices (30) for assigning specific control functions in respect of the devices (1, 1') or device groups (2) to the control signals, and
- information transmission devices (40) for transmitting the control signals from the foot-operated switches (10) to the devices (1, 1') or device groups (2),
**characterized in that**
the plug connectors (11, 12) comprise a data bus (21) which is formed in such a way that, after a large number of foot-operated switches (10) are connected to one another, the control signals of all the foot-operated switches (10) are available on the data bus (21), with each of the foot-operated switches (10) having two plug connectors (11, 12), which are connected to the data bus (21), in such a way that, when there is a series of foot-operated switches (10) which are connected to one another, the data bus (21) can be connected to the information transmission devices (40) at at least one of the plug connectors (11, 12).

2. Control device according to Claim 1,
**characterized in that**
the information transmission devices (40) comprise at least one transmitter (41), which can be connected to the foot-operated switches (10), and at least one receiver (42), which is connected to the devices (1, 1') or the device groups (2), for transmitting the control signals in a wireless manner.

3. Control device according to either of the preceding claims,
**characterized by**
coding devices (43) for the identification of foot-operated switches (10) by the devices (1, 1') or device groups (2).

## Revendications

1. Moyen de commande destiné à commander des dispositifs (1, 1') ou groupes de dispositifs (2) électriques médicaux, comprenant :
- au moins deux commutateurs au pied (10) destinés à générer des signaux de commande pour commander les dispositifs (1, 1') ou groupes de dispositifs (2),
- des connecteurs (11, 12) sur les commutateurs au pied (10), au moyen desquels les commutateurs au pied (10) peuvent être connectés les uns aux autres,
- des moyens d'affectation (30) destinés à affecter des fonctions de commande déterminées aux signaux de commande en fonction des dispositifs (1, 1') ou groupes de dispositifs (2), et
- des moyens de transmission d'informations (40) destinés à transmettre les signaux de commande des commutateurs au pied (10) aux dispositifs (1, 1') ou groupes de dispositifs (2),
**caractérisé en ce que** les connecteurs (11, 12) comprennent un bus de données (21) qui est conçu de telle manière qu'après connexion d'une pluralité de commutateurs au pied (10) les uns aux autres, les signaux de commande de tous les commutateurs au pied (10) sont fournis au bus de données (21),
dans lequel chacun des commutateurs au pied (10) comprend deux connecteurs (11, 12) connectés au bus de données (21) de telle manière que, dans le cas d'une série de commutateurs au pied (10) connectés les uns aux autres, le bus de données (21) puisse être connecté aux moyens de transmission d'informations (40) au niveau d'au moins l'un des connecteurs (11, 12).

2. Moyen de commande selon la revendication 1, **caractérisé en ce que** les moyens de transmission d'informations (40) comprennent au moins un émetteur (41) pouvant être connecté aux commutateurs au pied (10) et au moins un récepteur (42) connecté aux dispositifs (1, 1') ou groupes de dispositifs (2) pour la transmission sans fil des signaux de commande.

3. Moyen de commande selon l'une quelconque des revendications précédentes, **caractérisé par** des moyens de codage (43) destinés à identifier les commutateurs au pied (10) par l'intermédiaire des dispositifs (1, 1') ou groupes de dispositifs (2).
